# FASCICULE DE BREVET EUROPEEN

(11) **EP 0 733 636 B1**
(45) Date de publication et mention de la délivrance du brevet: **13.02.2002**
(21) Numéro de dépôt: 96480033.8
(22) Date de dépôt: 21.03.1996
(51) Int. Cl.: C07F 7/02, C07F 7/04, C12P 9/00

(54) **Extraction de composés organiques de silicium biologiquement actifs d'origine algale**
Extraktion von organischen Siliziumverbindungen mit biologischer Aktivität aus Algen
Extraction of organic silicon compounds with biological activity from algae

(30) Priorité: 22.03.1995 FR 9503577
(43) Date de publication de la demande: 25.09.1996
(73) Titulaire: EXSYMOL S.A.M., MC-98000 Monte Carlo (MC); ALGUES ET MER S.à.r.l., 29242 Ile d'Ouessant (FR); OCEALYS S.à.r.l., 29200 Brest (FR)
(72) Inventeur: Seguin, Marie-Christine, 98000 Monaco (MC); Franco, André, 06500 Menton (FR); Feno, Emile, 06000 Nice (FR); Bresdin, Fabienne, 29840 Landunvez (FR); Moigne, Jean-Yves, 29242 Ile d'Ouessant (FR)
(74) Mandataire: Bonneau, Gérard

(56) Documents cités:
- CHEMICAL ABSTRACTS, vol. 92, no. 11, 1980 Columbus, Ohio, US; abstract no. 90536f, XP002005162 & DRUGS FOOD SEA MYTH REALITY?, (INT. SYMP.), 1977, pages 171-180, P.N. KAUL ET AL:
- D. WERNER: "Drugs and Food from the sea. Myth or reality?", 1978, P.N. KAUL, C.J. SINDEMANN, UNIV. OKLAHOMA
- ALLINGER ET AL.: "Chimie Organique, Structure des Molécules", 1975, MAC GRAW HILL, PARIS

## Description

### Domaine technique

La présente invention concerne les composés à base de silicium biologiquement actifs et en particulier un procédé d'extraction de composés de silicium biologiquement actifs à partir d'algues, le procédé de localisation de tels composés dans des parties spécifiques des algues.

### Etat de la technique antérieure

Le silicium, élément très répandu dans la nature est généralement connu sous ses formes inorganiques naturelles telles que la silice et les silicates, ou encore sous la forme de polymères synthétiques, les silicones. Ces composés siliciés sont très peu ou pas solubles en milieu aqueux ce qui explique leur faible incidence au niveau des organismes vivants.

Les travaux des demandeurs ont montré que les composés siliciés pouvaient constituer une forme de silicium assimilable par l'organisme (par opposition au silicium minéral ou aux silicones) à condition de posséder la propriété d'exister en solution aqueuse sous forme d'oligomères solubles de faible poids moléculaire. En outre, une autre caractéristique nécessaire à l'activité de ces oligomères en solution aqueuse est de présenter de nombreuses fonctions Si-OH. Il ressort ainsi que les propriétés biologiques de ces composés siliciés assimilables par l'organisme, ne sont observées que s'ils forment en solution des oligomères solubles, résultant d'un enchaînement de liaisons siloxanes Si-O-Si, riches en fonctions Si-OH.

L'espèce chimique impliquée dans la plupart des mécanismes biologiques mentionnés ci-dessus est une forme soluble du silicium, l'acide silicique, de formule Si(OH)₄. Ce composé est décrit dans le document CHEMICAL ABSTRACT, vol. 92, no. 11 ; abstract no. 90536f. Mais, étant donnée sa très forte propension à se polycondenser pour former de la silice, ce composé doit être stabilisé immédiatement après extraction.

Une difficulté rencontrée par les demandeurs a été d'obtenir des composés à base de silicium dans lesquels le silicium est lié par des liaisons susceptibles d'être hydrolysées pour obtenir des composés biologiquement actifs possédant une ou plusieurs fonctions Si-OH. Ce type de composés peut évidemment être synthétisé mais il est plus avantageux de pouvoir extraire directement ces composés à partir des végétaux. Malheureusement, tous les végétaux ne contiennent pas ce type de composés à base de silicium.

### Exposé de l'invention

L'idée de base qui a prévalu dans la genèse de cette invention est fondée sur une double constatation; à savoir que d'une part, il y a équivalence qualitative entre le contenu minéral du liquide physiologique humain et le contenu de l'eau de mer, et d'autre part, il y a analogie des mécanismes de multiplication cellulaire entre les tissus embryonnaires humains et les tissus d'origine algale.

C'est pourquoi les buts principaux de l'invention sont de fournir des composés organiques de silicium biologiquement actifs d'origine algale, et de localiser précisément les parties des algues contenant lesdits composés organiques de silicium biologiquement actifs.

Encore un autre but de l'invention est de réaliser l'extraction à partir d'algues de composés organiques de silicium biologiquement actifs.

Encore un autre but de l'invention est de réaliser la culture d'algues à partir desquelles peuvent être extraits des composés de silicium biologiquement actifs.

"L'objet principal de l'invention est donc un composé organique de silicium à base d'acide silicique. Ce composé est un extrait d'origine algale provenant essentiellement des organes reproducteurs et des tissus jeunes et est stabilisé et biologiquement actif."

Un autre objet de l'invention est une méthode de localisation des parties d'algues à partir desquelles peut être extrait le composé selon l'invention comprenant les étapes suivantes effectuées à partir d'un échantillon desdites parties d'algues : fixation de l'échantillon dans un bloc de résine polymérisée, découpe de coupes fines dans le bloc de résine polymérisé, et micro-analyse d'une des coupes par spectrométrie pour détecter les parties contenant du silicium selon une concentration supérieure à un seuil prédéterminé.

Encore un autre objet de l'invention est une méthode d'extraction des composés organiques de silicium selon l'invention comprenant les étapes consistant à provoquer l'éclatement des cellules des parties d'algues contenant les composés à extraire, éliminer les épaississants par précipitation, stabiliser dans les composés organiques de silicium les liaisons Si-OH naturelles ou obtenues par hydrolyse des liaisons Si-OR', et purifier les concentrats résultant de la stabilisation par ultra-centrifugation.

### Description détaillée de l'invention

"Les demandeurs ont trouvé que certaines parties d'algues macroscopiques contenaient des composés organiques du silicium. Ces composés sont à base d'acide silicique biologiquement actif. ".

Une méthode de localisation originale décrite ci-dessous prouve que ces composés de silicium se trouvent principalement dans les organes reproducteurs et dans les tissus jeunes de certaines espèces d'algues.

### METHODE DE LOCALISATION

Généralement, les algues contiennent du silicium, que ce soit sous forme inorganique, ou sous forme de composés organiques. Ainsi, la présence de la forme inorganique du silicium est bien connue dans les algues unicellulaires du type diatomée dans lesquelles la carapace silicifiée est constituée par un dépôt extracellulaire de silicium. Mais les demandeurs ont découvert que les composés organiques selon l'invention se trouvaient essentiellement à l'intérieur des cellules d'algues bien que des composés de silicium non appropriés dans le cadre de l'invention aient été localisés dans le ciment intercellulaire.

Les composés organiques de silicium, les seuls intéressants dans le cadre de la présente invention, existent en quantité variable selon l'espèce d'algue, et ne se trouvent pas dans toutes les parties de l'algue. Ainsi, certaines espèces comme celles appartenant à la famille des algues rouges ou Rhodophycées semblent plus intéressantes que d'autres.

Il a donc été nécessaire d'élaborer une méthode pour localiser les parties d'algue, et déterminer quelles espèces doit on sélectionner pour en extraire des composés organiques de silicium selon la méthode qui vient d'être décrite.

Pour une variété d'algue donnée, une méthode de localisation préférée consiste à fixer des échantillons d'algues dans une résine, puis découper les échantillons fixés en coupes fines de façon à pouvoir effectuer une micro-analyse des tissus consistant en une spectrométrie aux rayons X suivie d'une analyse ionique.

De façon à fixer des échantillons dans la résine, la première étape consiste à mettre les échantillons dans un fixateur usuel tel que du glutaraldéhyde ou un mélange formol-glutaraldéhyde en solution dans l'eau de mer et sous un léger vide correspondant à une pression inférieure à 10⁻³ bar. Les échantillons fixés peuvent être éventuellement postfixés par du tétraoxyde d'osmium. Au cours de la deuxième étape, les échantillons sont déshydratés dans une série de bains à degré alcoolique croissant en utilisant de l'eau de mer pour la solution alcoolique (plutôt que de l'eau pure) dans le but de maintenir une pression osmotique égale à la pression osmotique du milieu naturel des cellules. Une troisième étape consiste à mettre les échantillons dans une résine thermodurcissable liquide sous un léger vide pour que, lorsque le vide disparait, il y ait une meilleure pénétration de l'alcool et de la résine à l'intérieur des cellules. Ceci permet d'obtenir petit à petit la substitution de l'alcool par la résine dans les échantillons, la résine utilisée étant de préférence une résine époxyde. Enfin, au cours d'une dernière étape, on procède à la polymérisation de la résine, notamment par chauffage à 70°C.

Les échantillons fixés sont alors découpés en coupes fines (0,7 µm (µ)) et semi-fines (2 µm (µ)) sur lesquelles on procède à la micro-analyse permettant de sélectionner précisément les parties d'algues contenant les composés organiques de silicium.

La micro-analyse des échantillons fixés débute par une spectrométrie aux rayons X permettant la détection du silicium dans les échantillons en utilisant des coupes fines (0,7 µm (µ)). Cette méthode permet d'identifier les éléments constitutifs du tissu biologique par leurs raies X caractéristiques. Pour ce faire, l'échantillon à analyser est déposé sur une grille de cuivre. L'échantillon est alors bombardé par un fin faisceau d'électrons d'une énergie comprise entre 0,1 et 50 Kev, appelé sonde électronique. Les spectres de rayonnement X émis par l'échantillon sont analysés par un spectromètre à sélection d'énergie. Les impulsions sont triées en fonction de leur énergie et un système expert interprète les résultats. Par cette méthode tous les éléments de numéro atomique supérieur à 4 sont détectés avec une limite de détection d'environ 10⁻¹⁴ à 10⁻¹⁵ g, ce qui correspond à une teneur inférieure à 100 ppm.

Si l'échantillon analysé comporte du silicium, on procède alors à une analyse ionique des ions secondaires permettant donc de visualiser les endroits de l'échantillon où se trouve le silicium. Pour cette analyse, dans la mesure où l'analyse ionique des électrons secondaires provoque l'érosion de la coupe, il est préférable d'utiliser des coupes semi-fines (2 µm (µ)) provenant d'échantillons qui ont été postfixés par du tétraoxyde d'osmium.

L'analyse ionique des ions secondaires est une méthode de microanalyse combinant la spectrométrie de masse et la microscopie corpusculaire. Dans la spectrométrie de masse, les molécules subissent une réaction chimique de cassure permettant de les ioniser, les ions étant ensuite séparés en fonction du rapport de masse sur charge (m/z) dans un système d'analyse. le mécanisme de l'émission ionique secondaire s'obtient après bombardement de l'échantillon par un faisceau d'ions primaires (le potentiel du faisceau primaire est ajustable jusqu'à 12 kV environ). Les ions primaires percutent les atomes de l'échantillon et ceux qui se trouvent au voisinage de la surface. Ces atomes, arrachés à l'échantillon, quittent celui-ci soit sous forme neutre, soit sous forme chargée (positivement ou négativement). Ces particules chargées appelées ions secondaires sont utilisées pour l'analyse. Les ions secondaires peuvent être monoatomiques ou polyatomiques. L'optique corpusculaire à focalisation bidirectionnelle permet d'obtenir une image ionique secondaire filtrée de la surface de l'échantillon solide. La particularité de cette méthode est qu'elle permet à la fois d'identifier tous les éléments de la classification périodique et d'obtenir des images de la distribution avec une résolution spatiale inférieure au micromètre.

Malheureusement, l'analyse par spectrométrie de masse amène des artefacts dûs au fait que le silicium peut être confondu avec un composé de même masse 28. Ce composé ne peut être que Al-H⁺ dans la mesure où les composés de même masse atomique, à savoir C₂H₄, N₂, CO, CNH₂, sont des constituants courants des composés biologiques et donnent donc une image diffuse sur l'ensemble de l'échantillon. Il faut donc également effectuer l'analyse ionique de l'aluminium (masse atomique 27) permettant de mettre en évidence les parties de l'échantillon contenant Al. Si le résultat obtenu est le même que celui obtenu lors de l'analyse du silicium, c'est donc qu'il s'agit du composé Al-H⁺ et non de silicium. L'échantillon est donc à rejeter. Par contre, s'il n'y a pas superposition des résultats, ceci signifie que la partie localisée lors de la micro-analyse contient véritablement du silicium.

La méthode de localisation ci-dessus permet ainsi la localisation du silicium dans les différentes parties d'une espèce donnée d'algue, et la mise en oeuvre d'un procédé de culture dirigé vers la production de ces parties de l'algue les plus intéressantes. Ainsi, dans l'espèce d'algue Asparagopsis Armata de la classe des Rhodophycées, on a pu localiser de façon importante les composés de silicium dans le cystocarpe (organe de reproduction) et dans le tissu apical (tissu jeune), alors que la localisation du silicium est faible au niveau du thalle et des rameaux anciens.

### METHODE D'EXTRACTION

La méthode de localisation qui a été décrite précédemment permet la sélection des espèces d'algues présentant un intérêt pour l'extraction des composés organiques de silicium selon l'invention, mais également une extraction sélective des composés organiques du silicium dans les parties de l'algue les plus intéressantes pour cette extraction.

Pour procéder à l'extraction des composés organiques de silicium, il est indispensable de faire éclater les parois cellulaires des algues. Deux méthodes peuvent être utilisées : le cryobroyage ou la méthode par ultra haute pression.

Le cryobroyage consiste à effectuer une congélation préalable des algues en plaques de 5 à 6 kg. Ces plaques sont convoyées dans un broyeur au sein duquel est injecté de l'azote liquide, ce qui permet d'obtenir en sortie un produit à -50°C. Ce produit est sous forme particulaire, très vacuolaire (les particules sont séparées par des espaces importants) et donc volumineux, le cryobroyage ayant entraîné un éclatement d'une proportion importante de cellules, et donc une libération du contenu intracellulaire. On doit noter que d'autres techniques peuvent être utilisées pour provoquer l'éclatement des cellules.

Dans la méthode d'éclatement des cellules par ultra haute pression, les algues sont soumises pendant 6 à 30 mn à des pressions comprises entre 4.000 et 10.000 bars. Ainsi, chaque cellule de l'algue est soumise à une haute pression isostatique et omnidirectionnelle qui entraîne son éclatement.

Une fois les parois cellulaires éclatées, il est indispensable de faire précipiter les épaississants de l'algue (par exemple les caraghénanes) par des agents du type ammonium ou par tout autre procédé.

Bien que les composés organiques de silicium soient en partie stabilisés par des stabilisants naturels présents dans l'algue, il est préférable de compléter cette stabilisation en utilisant de préférence lesdits stabilisants naturels. Cette stabilisation est due à la création de liaisons faibles (ponts hydrogène) empêchant la polycondensation qui ne manquerait pas de se produire à cause des liaisons Si-OH. Les stabilisants utilisés peuvent être des composés hydroxyacides carboxyliques et particulièrement les alpha et les bêta hydroxyacides, les glucuronides, des aminoacides hydroxylés ou phénoliques, des composés possédant plusieurs fonctions alcools (ou phénols). On peut citer dans cette catégorie les glycols, les catéchols et les catécholamines, les polyols tel que le glycérol, les monosaccharides, ou encore des acides phénoliques tels que l'acide gallique.

Après stabilisation, les concentrats de composés organiques de silicium sont enfin purifiés par ultra centrifugation, ou centrifugation à très grande vitesse et dialyse. Les dérivés de silicium autres que les composés organiques recherchés sont éliminés dans les culots de centrifugation. Dans le cas de l'espèce mentionnée ci-dessus, la solution obtenue par cette méthode d'extraction avait une teneur en silicium d'environ 0,3g/l.

### APPLICATIONS

De façon générale, les composés extraits par la méthode ci-dessus pourront être utilisés dans de nombreuses applications mettant en jeu les propriétés des formes de silicium organique, à savoir les propriétés thérapeutiques, diététiques ou cosmétiques découlant de leur activité anti-inflammatoire, hydratante, régénératrice, anti-dégénérescente, normalisatrice, lipolytique, stimulatrice métabolique, anti-radicalaire et anti-glycation, et de façon générale l'activité de stimulation des défenses de l'organisme.

Ainsi, les exemples donnés ci-après illustrent l'activité des composés organiques du silicium d'origine algale dans les domaines diététique (exemple 1) et cosmétique (exemples 2, 3 et 4).

### Exemple 1 (traitement diététique)

On a procédé à la réalisation de gélules. Une gélule contient 0,59 g de composé organique de silicium d'origine algale extrait selon les principes de l'invention sous forme pulvérulente (on a fait adsorber l'extrait sur une poudre polyamide), ce qui conduit à 0,1 mg de silicium par gélule.

L'administration a été de 6 à 9 gélules par jour. L'absorption journalière de silicium algal permet une remise en forme avec rééducation ou disparition des douleurs osseuses, le traitement devant se poursuivre plusieurs mois.

### Exemple 2 (activité régénératrice)

On a procédé à une culture de fibroblastes humains dans des microplaques. Le composé organique de silicium d'origine algale est distribué dans un milieu MEM à raison de 8 ml par litre dosé en silicium, avec 2,5% de SVF, deux séries témoins étant réalisées avec 10% de SVF. La prolifération cellulaire est suivie par le test colorimétrique au rouge neutre (Borenfreund 1984). La valeur d'absorption du colorant étant directement proportionnelle au nombre de cellules vivantes, la présence de silicium organique algal augmente la prolifération de 55%.

### Exemple 3 (activité hydratante)

On a réalisé un gel aqueux à 12% de composé organique de silicium d'origine algale obtenu selon l'invention. Ce gel a été appliqué sur l'avant-bras droit durant trois jours. L'effet hydratant a été mesuré par une méthode infrarouge (à transformée de Fourrier), le degré d'hydratation étant donné par le rapport des bandes amides C=O à 1650 cm⁻¹ aux bandes amides C-N à 1550 cm⁻¹. On a mesuré ce degré d'hydratation quotidiennement, du 4ème au 10ème jour, en comparaison avec un gel aqueux de référence appliqué sur l'avant-bras gauche. Le test a été fait sur 6 personnes. Les résultats moyens sont les suivants :
- à J+1 après application, on a un degré d'hydratation de 2,3 par rapport à un degré de 1,5 pour le témoin,
- à J+7, ce même degré d'hydratation est de 2,09 par rapport à 1,37 pour le témoin.

Ces résultats montrent bien l'effet immédiat, et surtout dans le temps, d'une activité hydratante biologique (eau liée) manifeste.

### Exemple 4 (activité lipolytique)

On a maintenu en survie des adipocytes humains. On prend 10 microlitres de composé organique de silicium d'origine algale (soit 30 mg par litre en Si) dilués dans 100 microlitres de solution à 0,01 M de NH₄HCO₃ complétés à 3 ml par un tampon de Krebs-Ringer.

L'activité lipolytique est quantifiée par la libération de glycérol dosé par méthode enzymatique exprimée en nanomoles de glycérol par gramme de lipides totaux. Le composé de silicium algal à la dose étudiée stimule la lipolyse de base entre 0 et 60 minutes. La stimulation par rapport au témoin est de 134%.

## Revendications

1. Composé à base d'acide silicique **caractérisé en ce qu'**il est un extrait d'origine algale provenant essentiellement des organes reproducteurs et des tissus jeunes et **en ce qu'**il est stabilisé et biologiquement actif.

2. Méthode de localisation des parties d'algues à partir desquelles peut être extrait le composé selon la revendication 1, comprenant les étapes suivantes effectuées à partir d'un échantillon desdites parties d'algues :
fixation dudit échantillon dans un bloc de résine polymérisée,
découpe de coupes fines dans ledit bloc de résine polymérisé, et
micro-analyse d'une desdites coupes par spectrométrie pour détecter les parties contenant du silicium selon une concentration supérieure à un seuil prédéterminé.

3. Méthode selon la revendication 2, dans laquelle ladite étape de fixation dudit échantillon d'algue dans un bloc de résine polymérisée comprend les étapes de :
fixer ledit échantillon au moyen d'un fixateur tel que le glutaraldéhyde et sous un léger vide,
déshydrater ledit échantillon fixé dans une série de bains à degré alcoolique croissant en utilisant de l'eau de mer pour la solution alcoolique,
mettre ledit échantillon déshydraté dans un résine thermodurcissable pour que ladite résine remplace petit à petit l'alcool dans ledit échantillon et, procéder à la polymérisation de la résine.

4. Méthode selon la revendication 2 ou 3, dans laquelle ladite étape de micro-analyse consiste à :
détecter si l'échantillon analysé comporte du silicium par spectrométrie aux rayons X,
effectuer une analyse ionique des ions secondaires pour visualiser les portions de l'échantillon où se trouve le silicium, et
éliminer lesdites portions de l'échantillon lorsque celles-ci sont les mêmes que celles obtenues par analyse ionique de l'aluminium contenu dans l'échantillon.

5. Méthode d'extraction de composés selon la revendication 1 consistant à :
provoquer l'éclatement des cellules des parties d'algues contenant lesdits composés,
éliminer les épaississants de l'algue par précipitation avec des agents du type amonium,
stabiliser dans lesdits composés organiques de silicium, les liaisons Si-OH naturelles ou obtenues par hydrolyse des liaisons Si-OR', et
purifier les concentrats résultant de la stabilisation par ultra-centrifugation.

6. Méthode d'extraction selon la revendication 5, dans laquelle l'éclatement des cellules des parties d'algues contenant lesdits composés organiques de silicium, se fait par cryobroyage à environ -50°C.

7. Méthode d'extraction selon la revendication 6, dans laquelle l'éclatement des cellules des parties d'algues contenant lesdits composés organiques de silicium, se fait par ultra haute pression entre 4000 et 10000 bars.

8. Application du composé selon la revendication 1, pour obtenir une composition thérapeutique, diététique ou cosmétique ayant une activité anti-inflammatoire, régénératrice, anti-dégénérescente, normalisatrice, lipolytique, stimulatrice métabolique, anti-radicalaire ou anti-glycation, ou de façon générale une activité de stimulation des défenses de l'organisme humain ou animal.

## Patentansprüche

1. Verbindung auf der Grundlage von Kieselsäure, **dadurch gekennzeichnet, dass** sie ein Algenextrakt ist, der im wesentlichen von den reproduzierenden Organen und den jungen Geweben stammt, und dass sie stabilisiert und biologisch aktiv ist.

2. Verfahren zur Lokalisierung der Algenteile, aus denen die Verbindung nach Anspruch 1 extrahiert werden kann, das die folgenden Schritte umfasst, die an einer Probe von diesen Algenteilen durchgeführt werden:
Fixierung der Probe in einem Block aus polymerisiertem Harz,
Ausschneiden von dünnen Schnitten in diesem Block aus polymerisiertem Harz und
Mikroanalyse eines dieser Schnitte durch Spektrometrie, um die Teile zu ermitteln, die Silicium in einer Konzentration enthalten, die über einer vorbestimmten Schwelle liegt.

3. Verfahren nach Anspruch 2, bei dem der Schritt der Fixierung der Algenprobe in einem Block aus polymerisiertem Harz die folgenden Schritt umfasst:
Fixierung der Probe mit Hilfe eines Fixiermittels wie Glutaraldehyd und unter einem leichten Unterdruck, Dehydratisierung der fixierten Probe in einer Reihe von Bädern mit steigendem Alkoholgehalt unter Verwendung von Meerwasser für die alkoholische Lösung,
Einführen der dehydratisierten Probe in ein wärmehärtendes Harz, so dass das Harz allmählich den Alkohol in der Probe ersetzt, und
Polymerisierung des Harzes.

4. Verfahren nach Anspruch 2 oder 3, bei dem der Schritt der Mikroanalyse darin besteht, dass
durch Röntgenspektrometrie ermittelt wird, ob die analysierte Probe Silicium enthält,
eine lonenanalyse der Sekundärionen durch Sichtbarmachen der Teile der Probe, in denen sich das Silicium befindet, durchgeführt wird und
diese Teile der Probe abgetrennt werden, wenn sie dieselben sind wie die, die durch Ionenanalyse des in der Probe enthaltenen Aluminiums erhalten wurden.

5. Verfahren zur Extraktion von Verbindungen nach Anspruch 1, das darin besteht, dass
das Aufplatzen der Zellen der diese Verbindungen enthaltenden Algenteile bewirkt wird,
die verdickenden Mittel der Alge durch Ausfällen mit Mitteln vom Typ Ammonium entfernt werden,
in diesen organischen Siliciumverbindungen die natürlichen oder durch Hydrolyse der Si-OR'-Bindungen erhaltenen Si-OH-Bindungen stabilisert werden und
die aus der Stabilisierung sich ergebenden Konzentrate durch Ultrazentrifugation gereinigt werden.

6. Extraktionsverfahren nach Anspruch 5, bei dem das Aufplatzen der Zellen der die organischen Siliciumverbindungen enthaltenden Algenteile durch Tieftemperatur-Zerkleinerung bei -50°C bewirkt wird.

7. Extraktionsverfahren nach Anspruch 6, bei dem das Aufplatzen der Zellen der die organischen Siliciumverbindungen enthaltenden Algenteile durch Ultrahochdruck zwischen 4 000 und 10 000 bar bewirkt wird.

8. Verwendung der Verbindung nach Anspruch 1 zur Herstellung einer therapeutischen, diätetischen oder kosmetischen Zusammensetzung mit entzündungshemmender, regenerierender, antidegenerierender, normalisierender, lipolytischer, den Stoffwechsel stimulierender Wirkung, mit Schutzwirkung gegen freie Radikale oder mit Antiglycationswirkung oder allgemein mit stimulierender Wirkung auf die Abwehrkräfte des menschlichen oder tierischen Organismus.

## Claims

1. Compound containing silicic acid **characterized in that** said compound is an extract of algal origin coming essentially from the reproductive organs and the young tissues of the alga and **in that** said compound is stabilized and biologically active.

2. Method of location of the algae parts from which can be extracted the compound according to claim 1, including the following steps performed on a sample of the said parts of the algae :
fixation of the said sample in a block of a polymerized resin,
slicing in fine cuts of the said block of a polymerized resin , and
elemental analysis of the said fine cuts by spectrometry in order to locate the parts of the algae having a content of silicon superior to an already chosen level.

3. Method according to claim 2, in which said fixation step of the said sample of alga in a polymerized resin includes the following steps:
fixation of the said sample by the means of a fixative such as glutaraldehyde, and under a slight vacuum
dehydration of the said fixed sample by successive bathing in a series of alcohol-containing bath of increasing degree of alcohol using sea water for the alcohol solution
placing said dehydrated sample in a thermohardening resin in such a way that said resin can replace slowly the alcohol contained in the said sample, and
proceed to the polymerization of the resin.

4. Method according to claim 2 or 3, in which the said step of elemental analysis consists in :
detect if the analysed sample contains some silicon using X ray spectrometry,
carry out an ionic analysis of the secondary ions in order to visualize the parts of the sample where silicon is located, and
withdraw the said parts from the rest of the sample when they are the same that the one identified by ionic analysis of aluminium.

5. Extraction procedure of the said compound according to the claim 1 consisting in:
induce the burst of the cells in the parts of the algae that contain the said compounds,
withdraw the thickening agents of the alga by selective precipitation using ammonium-type agents,
stabilize in the said organic silicon compound , the natural Si-OH linkages or linkages induced by the hydrolysis of the linkages Si-OR', and
purify the concentrates resulting of the stabilization procedure by ultra-centrifugation.

6. Extraction procedure according to the claim 5, in which the burst of the cells in the parts of the alga that contain the said organic silicon compound, is obtained by cryopounding at about -50°C.

7. Extraction procedure according to the claim 6, in which the burst of the cells in the parts of the alga that contain the said organic silicon compound, is obtained by ultra high pressure, comprised between 4000 and 10000 bars.

8. Application of the compound according to the claim 1, in order to obtain a therapeutical, dietary or cosmetic composition, having an anti-inflammatory, tissue regenerative, normalizing, lipolytic, metabolic stimulating, anti free radical or anti-glycation activity, or in a more general way an activity of stimulation of the defense mechanisms of the human or animal organisms.
